**Europäisches Patentamt**

**European Patent Office**

**Office européen des brevets**

(19)

(11) Numéro de publication : **0 386 854 B1**

(12)

# FASCICULE DE BREVET EUROPEEN

(45) Date de publication du fascicule du brevet :
**15.02.95 Bulletin 95/07**

(51) Int. Cl.$^6$ : **G01N 33/86,** G01N 33/543, G01N 33/80

(21) Numéro de dépôt : **90200840.8**

(22) Date de dépôt : **19.03.86**

(54) **Procédé de détermination immunologique d'un facteur de coagulation dans un échantillon.**

(30) Priorité : **19.03.85 FR 8504013**

(43) Date de publication de la demande :
**12.09.90 Bulletin 90/37**

(60) Numéro de publication de la demande initiale
en application de l'article 76 CBE : **0 217 845**

(45) Mention de la délivrance du brevet :
**15.02.95 Bulletin 95/07**

(84) Etats contractants désignés :
**AT BE CH DE FR GB IT LI LU NL SE**

(56) Documents cités :
**EP-A- 0 138 222**
**US-A- 3 502 437**
**US-A- 3 990 850**
**PATENT ABSTRACTS OF JAPAN, vol. 10, no.
327 (P-513)[2383], 07 novembre 1986** #
**PATENT ABSTRACTS OF JAPAN, vol. 9, no. 72
(P-345)[1795], 02 avril 1985; p. 57 P 345** #

(73) Titulaire : **LEE, Helen Hwai-an**
**3, route des Gatines**
**F-78190 Elancourt (FR)**
Titulaire : **CANAVAGGIO, Michel Etienne**
**25 bis, rue Jasmin**
**F-75016 Paris (FR)**

(72) Inventeur : **LEE, Helen Hwai-an**
**3, route des Gatines**
**F-78190 Elancourt (FR)**
Inventeur : **CANAVAGGIO, Michel Etienne**
**25 bis, rue Jasmin**
**F-75016 Paris (FR)**

(74) Mandataire : **Phélip, Bruno et al**
**c/o Cabinet Harlé & Phélip**
**21, rue de La Rochefoucauld**
**F-75009 Paris (FR)**

EP 0 386 854 B1

## Description

La présente invention concerne un procédé d'identification , et éventuellement de dosage, de substances biologiques .

Les procédés existants pour détecter des substances biologiques sont nombreux . Parmi ces procédés , les plus courants sont l'hémagglutination, les immunoessais fluorescents, enzymatiques ou radioactifs .

Les immunoessais mentionnés précédemment nécessitent la mise en oeuvre de produits de couplage artificiels tels que des conjugués anticorps-enzyme ou un marquage radioactif.

Pour illustrer l'état de la technique, on peut citer le document Patent Abstracts of Japan, Vol.9, N° 72 (p-345) (1795), 2 Avril 1985, page 57, page 345; et JP-A-59 203 957 SIGIURA SHINYAKU KAIHATSU KENYUSHO K.K. 19-11-1984.

Il est connu de ce document un réactif qui sert à mesurer l'activité de l'enzyme plasmatique kallikréine conjuguée à l'$\alpha_2$-macroglobuline dans le plasma sanguin, en présence d'un substrat synthétique. La technique d'immunoessai enzymatique utilisée nécessite la mise en oeuvre de produits de couplage artificiels anticorps-enzyme conjugués.

Un tel document ne décrit pas un procédé de détermination immunologique d'un facteur de coagulation, dans lequel la présence du facteur de coagulation est révélée à l'aide d'une propriété inhérente audit facteur, qui doit être activé avant l'étape de révélation.

Le brevet US-A-3.990.85O décrit un conditionnement particulier d'un test de groupage par la réaction classique d'agglutination en phase liquide; simplement, pour plus de commodité chez l'utilisateur (en particulier au lit du malade), les réactifs sont présents sous forme sèche sur une carte prête à l'emploi . Lorsque l'on rajoute une goutte de sang, l'immunosérum est réhydraté et entraîne une réaction d'agglutination classique.

Le brevet US 3.5O2.437 concerne une carte d'identification portable, utilisable pour le test de groupe sanguin et d'histocompatibilité entre un donneur et un receveur de sang, ou encore indiquant de façon permanente le résultat de ces tests , en vue d'une utilisation ultérieure. Les tests mettent en oeuvre des réactions d'hémagglutination classiques.

La présente invention a pour objet un procédé n'ayant pas recours à des produits de couplage artificiels ou à des produits radioactifs et qui puisse être mis en oeuvre en phase solide, à la différence de l'hémagglutination. Ce procédé présente, en particulier, l'avantage d'être simple, rapide et de pouvoir être automatisé tout en étant très sensible.

Plus particulièrement, la présente invention concerne un procédé de détermination d'un facteur de coagulation dans un échantillon, caractérisé en ce que:

a) on immobilise sur une phase solide un anticorps ayant une affinité de fixation pour ledit facteur de coagulation;

b) on met à incuber l'échantillon en présence de ladite phase solide sur laquelle est immobilisé l'anticorps;

c) on lave la phase solide après incubation; et

d) on révèle la présence dudit facteur de coagulation, fixé sur la phase solide par l'intermédiaire de l'anticorps à l'aide d'une propriété inhérente audit facteur de coagulation.

Le procédé selon la présente invention permet de mettre en évidence toutes les substances biologiques qui seront nommées ci-après "autorévélables ", c'est-à-dire tout produit biologique qui possède une propriété inhérente qui peut être mise directement ou indirectement en évidence pour l'homme ou la machine.

Les substances autorévélables sont, en particulier , les substances présentant une coloration naturelle , ou bien présentant une apparence particulière sous un rayonnement artificiel , ou encore les substances capables, directement lorsqu'il s'agit d'une macromolécule , ou grâce à certains éléments qu'elles contiennent, enzyme endogène ou composant chimique par exemple , de colorer un substrat ou de générateur une fluorescence ou une luminescence, protéine C par exemple .

Ce procédé est , en outre, particulièrement intéressant pour la détection de substances biologiques révélables indirectement par une coloration ou une réaction enzymatique endogène, en présence d'un substrat et éventuellement après activation enzymatique .

Les substances biologiques autorévélables selon l'invention sont des facteurs de la coagulation . Un exemple est donné ci-après avec la protéine C plasmatique .

La phase solide peut être n'importe quel support habituellement utilisé pour les immunoessais , qu'il s'agisse de support en forme de bille, de puits sur des plaques de microtitration, de bandelettes réactives ou autres . Ce support peut être en n'importe quel polymère d'origine naturelle ou synthétique ou en substance amorphe tel le verre. On utilise avantageusement pour les bandelettes réactives les membranes de nitrocellulose ou de nylon, pour les billes ou microplaques, les matières plastiques polyvinyl , polypropylène, polystyrène ou autre . On peut encore utiliser des gels ou des particules à base d'agarose , d'acrylamide ou de latex.

La sensibilisation de la phase solide par l'anticorps ayant une affinité vis-à-vis de la substance à doser est faite soit par adsorption passive, soit par couplage covalent selon la nature du support et des impératifs du test . La phase solide, après adsorption de l'anticorps, peut être saturée par des protéines ou macromolécules telles que l'albumine bovine , le sérum de veau foetal , la gélatine , ou chimiquement modifiée par des agents chimiques comme le Tween®, peut empêcher les interactions non spécifiques . Cette phase solide peut être modifiée pour être conservée sous forme desséchée ou lyophilisée.

Dans un mode de mise en oeuvre particulièrement intéressant de l'invention , la phase solide est constituée par une bandelette réactive ou une plaque sensibilisée par au moins deux anticorps distincts, spécifiques de différentes substances biologiques susceptibles d'être présentes dans l'échantillon à tester .

Les substances biologiques autorévélables sont mises à incuber en présence de la phase solide en milieu aqueux . Ce milieu peut être un liquide biologique normal ou pathologique, tel le sang , le sérum , le plasma , l'urine , le liquide céphalorachidien, un liquide d'épanchement plural , péritonéal , synovial ou autre , ou un milieu artificiel isotonique comportant éventuellement des substances d'optimisation ( protéines, sels, macromolécules, agents chimiques tel le Tween) dans lequel le prélèvement est resuspendu . L'incubation se fait à température ambiante ou modifiée avec ou sans agitation pendant un temps qui dépendra de la nature de la substance et de sa capacité à se fixer sur l'anticorps . Après incubation, la phase liquide est éliminée par séparation de phase ou lavage par exemple dans une solution telle un tampon PBS Tween à 0,05 %.

L'exemple ci-après est destiné à illustrer les caractéristiques et avantages du procédé selon la présente invention.

EXEMPLE :

Des anticorps monoclonaux purifiés anti Protéine C sont adsorbés passivement sur une plaque de microtitration de type plaque Microélisa M 129 B de la Société DYNATECH ou sur des barrettes sécables M 1798 A de la même Société .

Pour ce faire , les anticorps monoclonaux purifiés sont mis à incuber à la concentration optimum (5 à 10 µg/ml) dans du tampon bicarbonate 0,1 M pH 9,6, 3 heures à 37°C, à raison de 200 µl par puits, la plaque étant recouverte d'un film adhésif . Après lavage de la plaque en tampon PBS 0,15 M pH 7,4 , contenant du Tween® 20 à 0,1 %, les échantillons de plasma ou les dilutions de ceux-ci en tampon TBS Tris à 0,5 M NaCl 0,1 M, Albumine bovine 1%, pH 7,35 sont mis à incuber sous un volume de 200 µl pendant 30 minutes à 37°C.

La plaque est lavée par lavage automatique Multiwash Dynatech pendant 4 cycles par du tampon PBS 0,15 M pH 7,4 contenant du Tween® 20 à 0,05 %.

Après lavage, la protéine C immunoadsorbée est activée en présence de complexes thrombine humaine-Trombomoduline de lapin à 6 mM dans du tampon TBS 0,15 M, pH 7,35 + $CaCl_2$ 9mM . Cette solution d'activation est distribuée sous un volume de 200 µl et laissée à incuber 30 minutes à 37°C . Après lavage avec le même protocole que précédemment, 200 µl de substrat sont ajoutés dans chaque cupule (substrat 2366 de KABI Diagnostic ) à 1 mM dans du tampon Tris 0,05 M NaCl 0,15 M, pH 8,3).

La réaction est stoppée au bout d'une heure par l'addition d'acide acétique à une concentration finale de 15 %.

On a fait application du dosage de l'exemple à des plasmas normaux ou pathologiques.

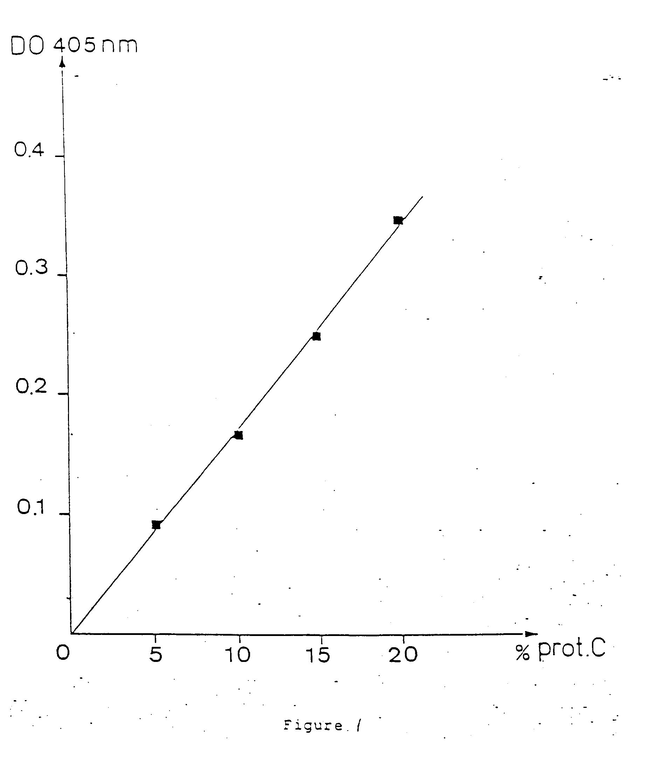

Un pool de 20 plasmas humains normaux est pris comme référence 100 % de protéine C . Quatre dilutions de cette référence correspondant aux concentrations en protéine C, 20 %, 15 % , 10%, 5% sont introduites dans le test pour obtenir la courbe de référence . Le témoin négatif 0% est constitué par un plasma préalablement débarrassé de protéine C par immunoadsorption . La courbe de référence est représentée ( figure ) . La figure est un diagramme sur lequel on a porté en abscisses les concentrations en protéine C exprimées en pourcentage d'un plasma normal et en ordonnées la densité optique DO lue à 405 nm.

Six groupes d'échantillons plasmatiques ont été testés afin d'évaluer la corrélation clinique du test de la présente invention comparativement à un test ELISA sandwich classique de dosage quantitatif .

Les résultats sont présentés dans le tableau .

Les résultats obtenus avec le test de la présente invention sont étroitement corrélés à la clinique et aux résultats quantitatifs du test ELISA. Le test selon l'invention permet en outre de dépister les déficits qualitatifs en protéine C ( altération de l'activité biologique de l'enzyme sans déficit quantitatif ) que le dosage ELISA ne peut pas mettre en évidence puisque ce dernier test est seulement quantitatif .

Le procédé de l'invention peut encore être mis en oeuvre avec des variantes.

Ainsi la protéine C plasmatique peut être activée avant distribution des échantillons dans les puits de microtitration ou encore au cours de la première incubation des échantillons . Dans ce cas , le substrat est directement ajouté après lavage de la phase solide . Egalement, l'activation peut se faire avec de la thrombine seule ou avec d'autres enzymes activateurs et en particulier certains enzymes extraits de venin de serpent (par exemple le venin RVVX de la Société SIGMA) . Il est encore possible d'utiliser un substrat fluorescent.

TABLEAU

| Groupe étudié | Nombre de plasmas | Concentration moyenne en protéine C par ELISA | Concentration moyenne en protéine C par le procédé de l'invention |
|---|---|---|---|
| I Sujets normaux | 5 | 98% | 91% |
| II Sujets sous anti vitamine K | 5 | 15% | 13% |
| III Déficits congénitaux en protéine C | 7 | 37% | 44% |
| IV Déficits congénitaux en protéine C + anti vitamine K | 8 | 11% | 14% |
| V Déficits congénitaux en facteurs de la coagulation autres que la protéine C | 6 | 92% | 97% |
| VI Déficit qualitatif en protéine C | 2 | 105% | 17% |

## Revendications

1. Procédé de détermination d'un facteur de coagulation dans un échantillon, caractérisé par le fait que :
   (a) on immobilise, sur une phase solide, un anticorps ayant une affinité de fixation pour ledit facteur de coagulation;
   (b) on met à incuber l'échantillon en présence de ladite phase solide sur laquelle est immobilisé l'anticorps;
   (c) on lave la phase solide après incubation;
   (d) on révèle l'activité enzymatique dudit facteur de coagulation, fixé sur la phase solide par l'intermé-

4

diaire de l'anticorps, en présence d'un substrat chromogénique ou fluorescent ,
ledit facteur ayant été activé avant l'étape de révélation.

**2.** Procédé selon la revendication 1, caractérisé par le fait que le facteur de coagulation à doser est la protéine C plasmatique .

**3.** Phase solide pour la mise en oeuvre du procédé selon l'une des revendications 1 ou 2, caractérisée par le fait qu'elle comporte plusieurs anticorps fixés sous forme sèche.

**4.** Phase solide selon la revendication 3, caractérisée par le fait qu'elle est sous forme de bandelettes ou de plaque de micro-titration ou de billes.

## Claims

**1.** Method for determining a clotting factor in a sample, characterized in that :
a) an antibody having a binding affinity for the said clotting factor is immobilized on a solid phase;
b) the sample is incubated in the presence of the said solid phase on which the antibody is immobilized;
c) the solid phase is washed after incubation;
d) the enzymatic activity of the said clotting factor, bound to the solid phase through the antibody, is revealed in the presence of a chromogenic or fluorescent substrate,
said factor having been activated before the revelation step.

**2.** Method according to claim 1 characterized in that the clotting factor to be determined is the plasmatic protein C.

**3.** Solid phase for carrying out the method according to one of claims 1 or 2 characterized in that it contains several antibodies bound in dry form.

**4.** Solid phase according to claim 3, characterized in that it is in the form of strips or micro-titration plates or beads.

## Patentansprüche

**1.** Verfahren zur Bestimmung des Gerinnungsfaktors in einer Probe, dadurch gekennzeichnet, daß
(a) ein eine Fixierungsaffinität für den Gerinnungsfaktor aufweisender Antikörper auf einem Feststoff aufgebracht wird,
(b) die Probe in Gegenwart des Feststoffs, auf dem der Antikörper aufgebracht ist, inkubiert wird,
(c) der Feststoff nach erfolgter Inkubation gewaschen wird,
(d) die enzymatische Aktivität des Gerinnungsfaktors, der auf dem Feststoff mittels des Antikörpers fixiert ist, in Gegenwart eines farbgebenden oder fluoreszierenden Substrats entwickelt wird,
wobei der Faktor vor der Entwicklungsstufe aktiviert worden ist.

**2.** Verfahren nach Anspruch 1, dadurch gekennzeichnet, daß der zu dosierende Gerinnungsfaktor C-Plasma-Protein ist.

**3.** Feststoff zur Anwendung des Verfahrens nach Anspruch 1 oder 2, dadurch gekennzeichnet, daß er mehrere auf ihm fixierte Antikörper in getrocknetem Zustand aufweist.

**4.** Feststoff nach Anspruch 3, dadurch gekennzeichnet, daß er in Form von Bändchen oder Mikrotitrierplatten oder Kugeln vorliegt.

Figure. 1